# EUROPÄISCHE PATENTANMELDUNG

(11) **EP 0 903 114 A1**
(43) Veröffentlichungstag der Anmeldung: **24.03.1999**
(21) Anmeldenummer: 98117183.8
(22) Anmeldetag: 10.09.1998
(51) Int. Cl.: A61B 19/08, A61B 19/02

(54) **Tischtuch für einen OP-Beistelltisch mit darin eingeschlagenen Operations-Set-Komponenten**

(30) Priorität: 15.09.1997 DE 19740600
(71) Anmelder: GFM Igenieurs- und Produktionstechnik GmbH, 45721 Haltern (DE)
(72) Erfinder: Gawrecki, Herbert, Dipl-Ing., 45721 Haltern (DE)
(74) Vertreter: Eichelbaum, Lambert, Dipl.-Ing.

(57) **Zusammenfassung**

Die Erfindung betrifft ein Tischtuch (1) (back table cover) für einen OP-Beistelltisch mit darin eingeschlagenen, unterschiedlichen Operations-Set-Komponenten (2), wie mayo-stand-covers, OP-Abdecktüchern, Klebestreifen, Handtüchern, Strümpfen oder dergleichen, welches von einer Verpackung (26) umhüllt ist.

Der Erfindung liegt die Aufgabe zugrunde, ein Tischtuch der eingangs genannten Art mit einer rasch produzierbaren Vakuumverpackung (26) zu schaffen, welcher das Tischtuch (1) in mechanisch und hygienisch einwandfreiem Zustand mit seinen Operations-Set-Komponenten (2) entnommen werden kann.

Diese Aufgabe wird erfindungsgemäß dadurch gelöst, daß das zusammengefaltete Tischtuch (1) an mindestens einer seiner Außenbereiche (18, 18a) mit einer zu seinem Innenraum (15) führenden, luftdurchlässigen Zone (17, 20, 29, 30, 34) versehen ist.

## Beschreibung

Die Erfindung betrifft ein Tischtuch (back table cover) für einen OP-Beistelltisch mit darin eingeschlagenen, unterschiedlichen Operations-Set-Komponenten, wie mayo-stand-covers, OP-Abdecktüchern, Klebestreifen, Handtüchern, Strümpfen oder dergleichen, welches von einer Verpackung umhüllt ist.

Ein durch offenkundige Vorbenutzung bekanntes Tischtuch der vorgenannten Art wird in einem OP-Saal für einen OP-Beistelltisch im Hintergrund gebraucht, auf welchem das sterile Tischtuch aus einer mit einem Peel-Verschluß versehenen Verpackung entnommen und derart auseinandergefaltet wird, daß es einerseits den gesamten Tisch bedeckt und nach der Entfaltung die darin eingeschlagenen Operations-Set-Komponenten, die gleichfalls sterilisiert sind, für den Operationsgebrauch entnommen werden können. Diese Operations-Set-Komponenten sind den jeweiligen spezifischen Operationen angepaßt und können daher höchst unterschiedlich sein. Das Tischtuch hingegen weist stets bestimmte Normmaße auf.

Die das Tischtuch mit den darin eingeschlagenen Operations-Set-Komponenten umhüllende Verpackung besteht nach dem Stand der Technik aus einer tiefgezogenen Kunststoffolie mit einem deckelartigen Abzieh-Verschluß (Peel-Verschluß, der von der bereits eine sterile Schleuse passierten Operationsschwester geöffnet und sodann das Tischtuch mit seinem eingeschlossenen Inhalt aus der Verpackung entnommen wird. Die bisherigen Verpackungen derartiger Tischtücher enthalten Luft und sind daher auch entsprechend voluminös.

Der wesentliche Nachteil der bisherigen Verpackungen dieser Tischtücher beruht jedoch darin, daß eine Undichtigkeit und damit eine Unsterilität der Verpackung und seines Inhaltes allenfalls zufällig, jedoch nicht ad hoc erkannt werden kann. Da derartige Tischtücher erst während der häufig kurzfristigen Operationsvorbereitungen aus der Verpackung entnommen werden, verzögert die Feststellung einer unsterilen Verpackung nicht nur die Vorbereitungen, sondern auch die Operation selbst, da eine neue Verpackung erst durch eine entsprechende sterile Schleuse herbeigeschafft werden muß.

Ein Tischtuch in einer kleineren Verpackung, dessen Undichtigkeit auch sofort vom Krankenhauspersonal wahrnehmbar ist, würde beispielsweise durch eine Vakuumverpackung geschaffen werden können. Da jedoch aus wirtschaftlichen Gründen die Fertigung eines derartigen Tischtuches mit seiner Verpackung an eine bestimmte Stückzahl pro Minute gebunden ist, käme hier nur eine sehr rasch wirkende Hochvakuumverpackung in Betracht. Wie jedoch erste Versuche gezeigt haben, wird dabei das Tischtuch bei der Absaugung der Luft aus seinem Innenraum mit den darin eingeschlagenen Operations-Set-Komponenten wie ein Luftballon aufgeblasen und dabei ganz oder teilweise zerstört.

Hier setzt nun die Erfindung ein. Dieser liegt die Aufgabe zugrunde, ein Tischtuch der eingangs genannten Art in einer rasch produzierbaren Vakuumverpackung zu schaffen, welches dieser in mechanisch und hygienisch einwandfreiem Zustand mit seinen Operations-Set-Komponenten entnommen werden kann.

Diese Aufgabe wird erfindungsgemäß dadurch gelöst, daß das zusammengefaltete Tischtuch an mindestens einer seiner Außenbereiche mit einer zum Innenraum führenden, luftdurchlässigen Zone versehen ist. Wie sich überraschend herausgestellt hat, kann ein derartig gestaltetes Tischtuch ohne Beschädigung seiner selbst bzw. seiner darin eingeschlagenen unterschiedlichen Operations-Set-Komponenten einwandfrei sowie sehr rasch mit einem Niederdruck-Evakuierungsverfahren in einer rasch und stark evakuierten Verpackung hygienisch sicher eingebracht werden.

Bei der Ausgestaltung der luftdurchlässigen Zone gestattet die Erfindung unterschiedliche Ausführungsformen:

Nach einer ersten Ausführungsform ist die luftdurchlässige Zone an einer Faltkante des Tischtuches vorgesehen. Diese Ausführung ermöglicht einen sehr breitflächigen, in den Innenraum des eingeschlagenen Tischtuches führenden Kanal.

Nach einer zweiten Ausführungsform ist die luftdurchlässige Zone an einer Außenseite des Tischtuches angeordnet. Auch diese Ausführung ermöglicht die Anordnung großer, zum Innenraum des Tischtuches führender Kanäle, so daß eine Luftevakuierung auch in entsprechend kurzer Zeiteinheit vorgenommen werden kann.

Nach einer dritten Ausführungsform ist die luftdurchlässige Zone zwischen zwei Faltflächen des Tischtuches vorgesehen. Dabei kann die luftdurchlässige Zone nach einer vorteilhaften Weiterbildung entweder von einer zwischen zwei Faltflächen eingelegten, bis in den Innenraum des gefalteten Tischtuches reichenden Schlauchleitung oder von einem von Abstandshaltern zwischen den Faltlagen geformten und gleichfalls bis in den Innenraum des Tischtuches führenden Kanal gebildet sein.

Nach einer ersten konkreten Ausführungsform besteht die luftdurchlässige Zone aus in das Tischtuch eingebrachten Schlitzen, sei es in der Nähe einer Faltkante oder sei es an einer Außenseite des Tischtuches. Diese Schlitze sind von einer Kunststoffolie abgedeckt. Dabei ist die Kunststoffolie vorteilhaft an den beiden Schlitzenden eines jeden Schlitzes sowie an der dem Innenbereich des Tischtuches zugeordneten Schlitzlängsseite mit dem Tischtuch verklebt, verschweißt oder durch eine Prägung verbunden. Dadurch wird ein vom Innenraum gezielt und mit geringen Strömungsverlusten verbundener Weg des Evakuierung-Luftstromes in Richtung auf die umhüllende Verpackung geschaffen, dessen Umlenkungswinkel in der Nähe der Schlitze 90° nicht übersteigt.

Nach einer weiteren konkreten Ausführungsform besteht die luftdurchlässige Zone aus einer in eine Außenseite des gefalteten Tischtuches eingebrachten oder aus einer bis zu einer Faltkante reichenden Öffnung, die von einer Membran verschlossen ist, welche von innen nach außen und umgekehrt luftdurchlässig, hingegen in umgekehrter Richtung flüssig- und feststoffdicht ist. Die Membran kann aus einem Tyvek- oder einem anderen Vliesstoff oder einem Tissue-Material bestehen. In diesem Fall bildet die Membran ein Diaphragma, welches die gewünschte Luft von innen nach außen und umgekehrt durchläßt, hingegen in umgekehrter Richtung das Eindringen von flüssigen und festen Stoffen sperrt.

Diese Membran ist vorteilhaft an ihren Außenrandbereichen dicht mit dem Rand der Öffnung des Tischtuches verklebt, verschweißt oder verprägt. "Dicht" heißt in diesem Fall in beiden Richtungen verschließend.

Bei den vorher beschriebenen Beispielen war die luftdurchlässige Zone stets direkt in dem Tischtuch angebracht. Die Erfindung eröffnet jedoch auch die Möglichkeit, eine luftdurchlässige Zone ohne Änderung des Tischtuches zu schaffen. So besteht nach einer besonders vorteilhaften Weiterbildung der Erfindung die luftdurchlässige Zone aus einem Luftkanal, der entweder von einer eingelegten, bis in den Innenraum des gefalteten Tischtuches reichenden Schlauchleitung oder von einem von Abstandshaltern zwischen zwei Faltlagen geformten und bis in den Innenraum führenden Kanal gebildet ist. Als Abstandshalter sind eingelegte, stangenartige Drittkörper vorgesehen, die mit der Verpackung entfernt werden.

Nach einer weiteren, vorteilhaften Ausgestaltung der Erfindung besteht die luftdurchlässige Zone aus einem Luftkanal, der von mindestens einer Unterbrechung einer zu einer Längsseite des Tischtuches parallel verlaufenden Klebeschicht in Verbindung mit den an die Unterbrechnung angrenzenden Flächen der jeweiligen Teile des Tischtuches gebildet ist. Dabei ist die Klebeschicht des Tischtuches vorteilhaft zwischen zwei getrennten und von der Klebeschicht wiederverbundenen Kunststoffolien-Teilen angeordnet, weil sich der Kunststoff des Tischtuches, hier in aller Regel Polyethylen, im Gegensatz zu dem darauf befindlichen Tissue problemlos kleben läßt.

Mehrere Ausführungsbeispiele der Erfindung sind in den Zeichnungen dargestellt. Dabei zeigen:
Fig. 1 die perspektivische Draufsicht auf ein noch ungefaltetes Tischtuch (back table cover) für einen OP-Beistelltisch mit darauf angeordneten, darin einzuschlagenden, unterschiedlichen Operations-Set-Komponenten,
Fig. 2 die perspektivische Draufsicht auf den Gegenstand von Fig. 1 nach dem Längseinschlag in Richtung der Pfeile von Fig. 1,
Fig. 3 das zusammengefaltete Tischtuch von Fig. 2 nach seiner Quereinschlagung in Richtung der Pfeile von Fig. 2,
Fig. 4 das Tischtuch mit seinen eingeschlagenen Operations-Set-Komponenten von Fig. 3 in einer Vakuumverpackung,
Fig. 5 die Schnittansicht entlang der Linie V/V von Fig. 2 in einer gegenüber dieser Fig. 2 vergrößerten Ansicht,
Fig. 6 die Schnittansicht entlang der Linie VI/VI von Fig. 3 in einer gegenüber dieser Fig. 3 vergrößerten Ansicht,
Fig. 7 die perspektivische Draufsicht auf ein zusammengefaltetes Tischtuch mit seinen darin eingeschlagenen Operations-Set-Komponenten gemäß Fig. 3 mit einer Membran an einer Außenseite zum Luftdurchlaß während des Evakuierungsvorganges,
Fig. 8 die Ausschnittsvergrößerung VIII von Fig. 7,
Fig. 9 die Schnittansicht IX/IX von Fig. 8,
Fig. 10 eine der Fig. 7 entsprechende perspektivische Draufsicht mit mehreren an einer Außenseite des Tischtuches angebrachten sowie von einer Folie abgedeckten Schlitzen für den Luftdurchlaß während des Evakuierungsprozesses,
Fig. 11 die Ausschnittsvergrößerung XI von Fig. 10,
Fig. 12 die Schnittansicht entlang der Linie XII/XII von Fig. 11.
Fig.13 bis 15 eine weitere Ausführungsform einer luftdurchlässigen Zone in Form einer zwischen den einander gegenüberliegenden Flächen zweier Faltlagen eingelegten Schlauchleitung, wobei die Fig. 13 und 14 den Fig. 5 und 6 entsprechen und Fig. 15 eine Ansicht in Richtung des Pfeiles XV von Fig. 14 ist,
Fig. 16 bis 18 eine weitere Ausführungsform einer luftdurchlässigen Zone in Form von Abstandshaltern zwischen den zwei Flächen zweier Faltlagen und einem somit geschaffenen, bis in den Innenraum führenden Kanal; dabei entspricht Fig. 16 der Fig. 6 und Fig. 17 der Fig. 5, während Fig. 18 die Ansicht in Richtung des Pfeiles 18 von Fig. 17 darstellt,
Fig. 19 die perspektivische Draufsicht auf eine weitere Ausführungsform eines noch ungefalteten Tischtuches mit den darin einzuschlagenden Operations-Set-Komponenten und einer Klebeschicht mit Unterbrechungen zur Schaffung von Strömungskanälen vom Innenraum nach Außen,
Fig. 20 die Ausschnittsvergrößerung XX von Fig. 19,
Fig. 21 die Ansicht des Tischtuches von Fig. 19 nach dem Falteinschlag seiner Längsseiten und
Fig. 22 die Ausschnittsvergrößerung XXII von Fig. 21.

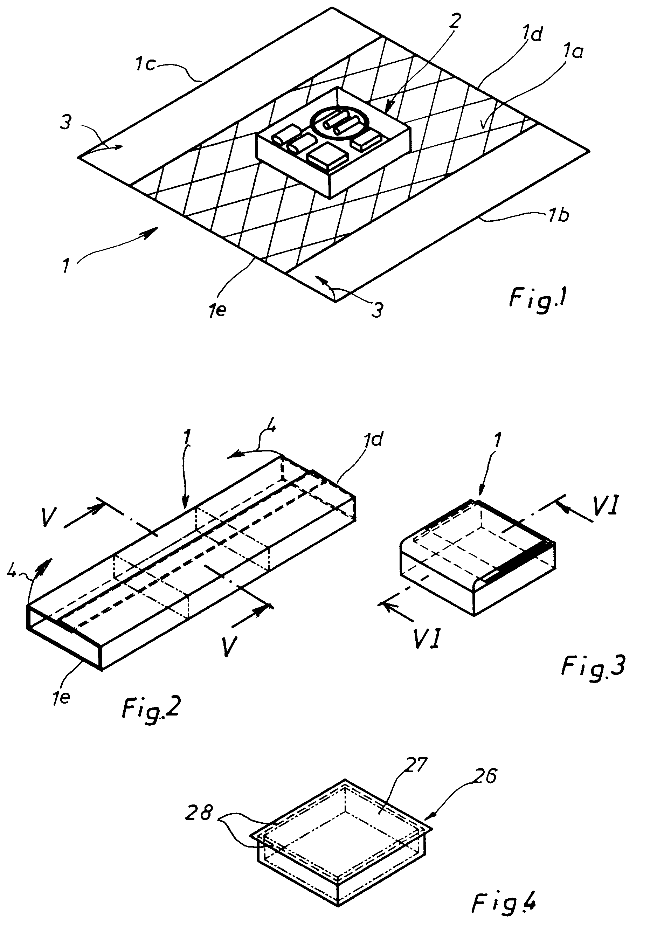

Gemäß den Fig. 1 bis 4 besteht das Tischtuch 1 aus einer grüneingefärbten Polyethylenfolie mit einem darauf rutschfest angeordneten saugfähigen Vliesstoff 1a gleicher Farbe sowie aus in dem Mittenbereich aufgelegten, im Tischtuch 1 einzuschlagenden Operations-Set-Komponenten 2. Diese Operations-Set-Komponenten 2 können nach der Art der Operation aus völlig unterschiedlichen Einzelteilen, wie mayo-stand-covers, OP-Abdecktücher, Klebestreifen, Handtücher, Strümpfe oder dergleichen, bestehen, die für die jeweils anstehende Operation benötigt werden.

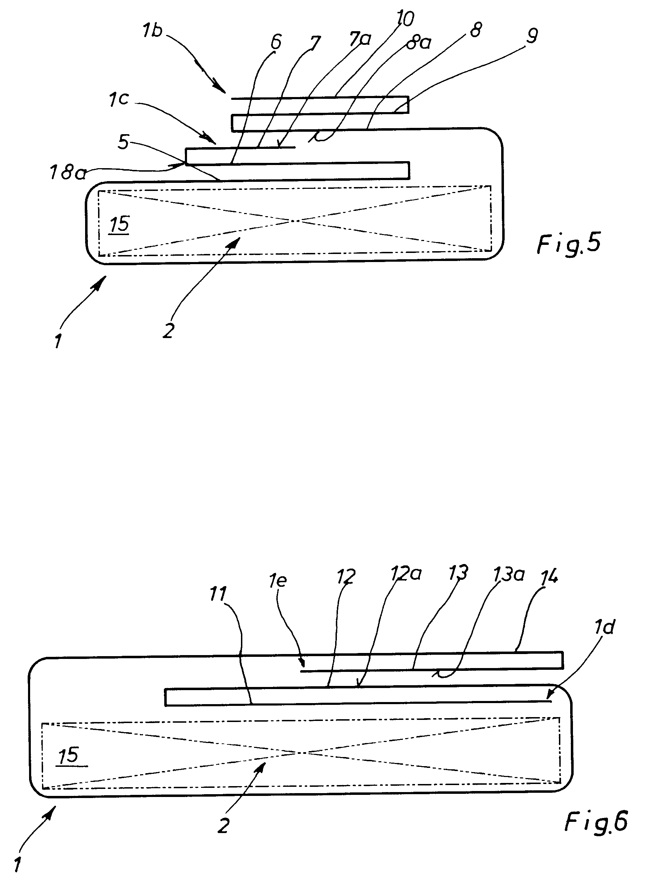

In Fig. 2 ist der Einschlag der Längsseiten 1b und 1c des Tischtuches 1 in Richtung der Pfeile 3 von Fig. 1 vorgenommen worden und im Querschnitt aus Fig. 5 mit den einzelnen Faltlagen ersichtlich. Dabei weist die linke Einschlagseite 1c drei Faltlagen 5, 6 und 7 und die rechte Einschlagseite 1b gleichfalls drei Faltlagen 8, 9 und 10 auf. Hiernach werden die Schmalseiten 1d und 1e von Fig. 2 in Richtung der Pfeile 4 eingeschlagen. Dieser Einschlag ist im Querschnitt in Fig. 6 dargestellt. Dabei weist die innenliegende Schmalseite 1d die Faltlagen 11 und 12 und die außenliegende Schmalseite 1e die Faltlagen 13 und 14 auf.

Wie aus den Fig. 5 und 6 entnehmbar ist, bilden die Faltlagen 7 und 8 gemäß Fig. 5 miteinander Kontaktflächen 7a und 8a und ebenso bilden gemäß Fig. 6 die einander zugekehrten Faltlagen 12 und 13 Kontaktflächen 12a und 13a, die beim Evakuieren der Luft aus dem Innenraum 15 des eingeschlagenen Tischtuches 1 derart miteinander "verkleben", daß die aus den Operations-Set-Komponenten zu entziehende Luft während des Evakuierungsvorganges das durch "Verklebung" nunmehr geschlossene Tischtuch 1 aufbläht. Dieser Aufblähvorgang führt nicht nur zur Beschädigung des Tischtuches 1 selbst, sondern auch der Operations-Set-Komponenten 2. Um dies zu vermeiden, sieht die Erfindung vor, daß das zusammengefaltete Tischtuch 1 an mindestens einer seiner Außenbereiche mit einer zum Innenraum 15 führenden, luftdurchlässigen Zone versehen ist.

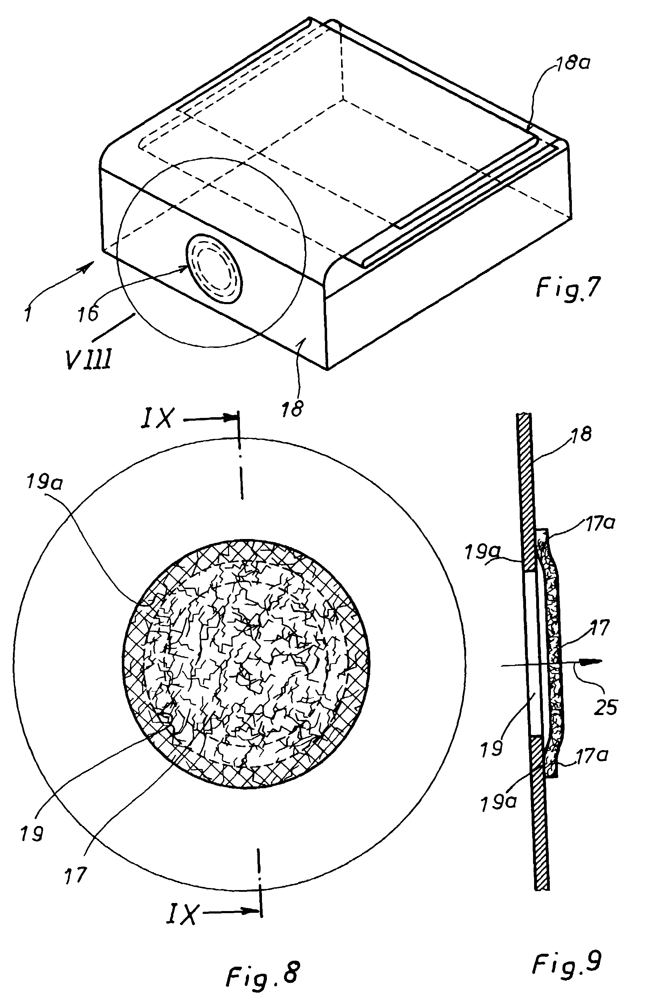

Nach einer ersten Ausführungsform gemäß den Fig. 7 bis 9 besteht diese luftdurchlässige Zone 16 aus einer Membran 17 aus Tyvek- oder einem anderen Vliesstoff oder aus einem Tissue-Material, welche gemäß Fig. 7 in einer Außenseite 18 des gefalteten Tischtuches 1 angeordnet ist. Wie aus den Fig. 8 und 9 entnommen werden kann, ist in dieser Außenseite 18 eine Öffnung 19 in dem Tischtuch 1 angeordnet, die von der Membran 17 verschlossen ist. In ihrem Außenrandbereich 17a ist die Membran 17 dicht mit dem Rand 19a der Öffnung 19 des Tischtuches 1 entweder verklebt, verschweißt oder verprägt. "Dicht" heißt hier abschließend zu beiden Seiten.

Es versteht sich, daß die luftdurchlässige Zone 16 auch in einer Faltkante 18a (siehe Fig. 5, 13, 14, 16 und 17) angeordnet werden kann.

Auf diese Weise wird die Funktion der technischen Membran 17 als Diaphragma sichergestellt, welche beim Evakuierungsvorgang zwar Luft vom Innenraum 15 nach außen und umgekehrt durchläßt, hingegen den Eintritt von Feuchtigkeit oder Feststoffen in den Innenraum 15 unterbindet.

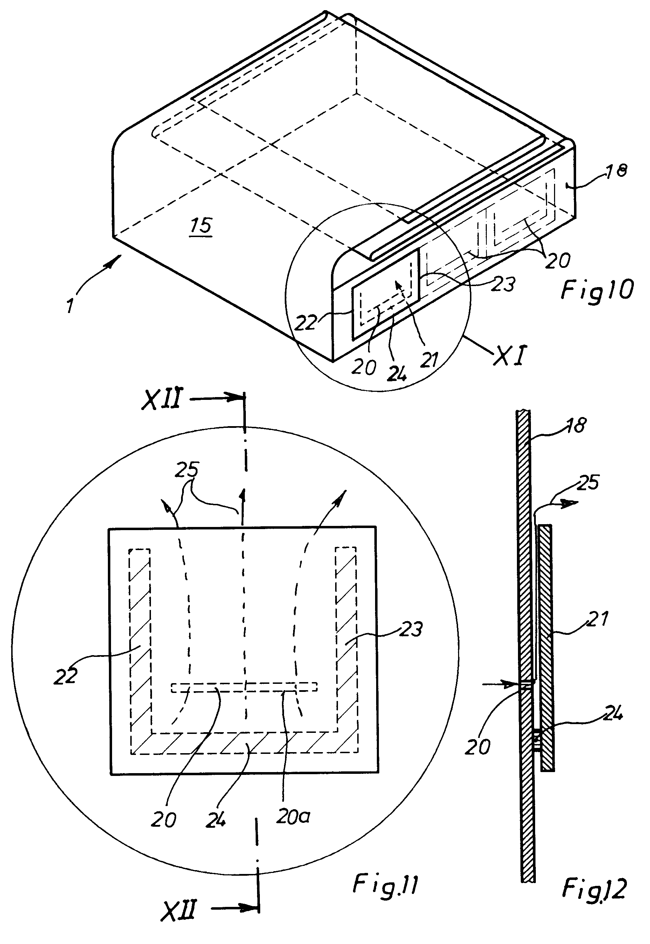

In den Fig. 10 bis 12 besteht die luftdurchlässige Zone aus mehereren Schlitzen 20, die in einer Außenfläche 18 des zusammengefalteten Tischtuches 1 angeordnet sind. Diese Schlitze 20 werden von einer Kunststoffolie 21 derart abgedeckt, daß die Kunststoffolie an den Seiten 22, 23 und an der dem Innenbereich zugeordneten Schlitzlängsseite 20a bei 24 mit der Polyethylenfolie des Tischtuches 1 verschweißt, verklebt oder verprägt ist. Dadurch kann die Luft aus dem Innenraum 15 des gefalteten Tischtuches 1 durch die Schlitze 20 in Richtung der Pfeile 25 zu der umhüllenden Verpackung 26 gemäß Fig. 4 austreten. Der Deckel 27 dieser Verpackung 26 ist mittels eines Abziehverschlusses 28 (Peel-Verschluß) mit der Verpackung 26 verbunden. Die Pfeile 25 zeigen auch in den Fig. 9, 14, 17, 18 und 21 den Strömungsweg der Luft vom Innenraum 15 nach außen.

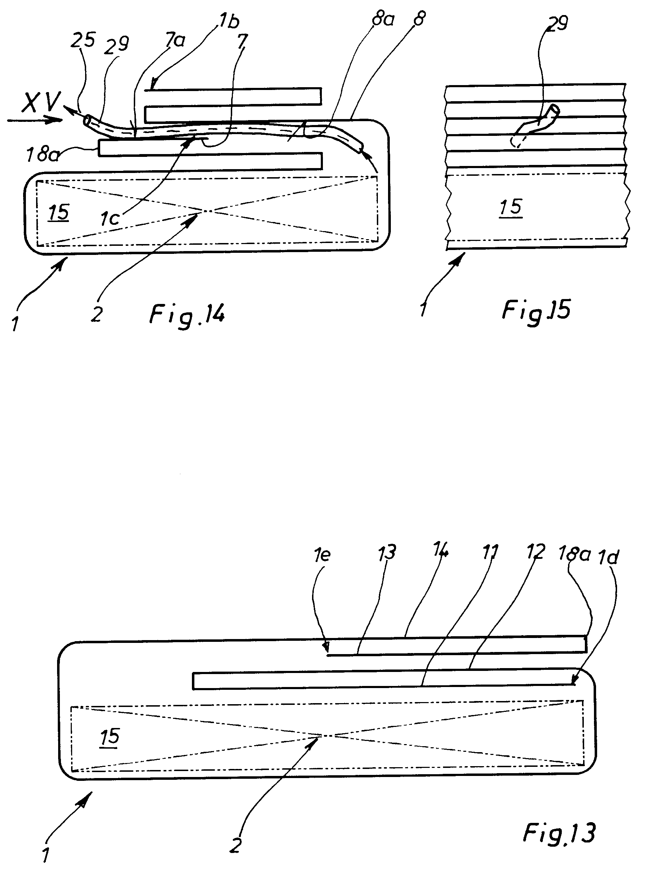

In den Fig. 13 bis 15 besteht die luftdurchlässige Zone aus einem Luftkanal in Form einer Schlauchleitung 29, die zwischen der Kontaktfläche 7a der Faltlage 7 und der Kontaktfläche 8a der Faltlage 8 (siehe auch Fig. 5) eingelegt ist und bis in den Innenraum 15 des zusammengefalteten Tischtuches 1 führt. Da diese Schlauchleitung 29 lose zwischen den Faltlagen 7 und 8 angeordnet ist, kann sie problemlos beim Herausnehmen des zusammengefalteten Tischtuches 1 aus der Verpackung 26 mit letzterer entfernt werden.

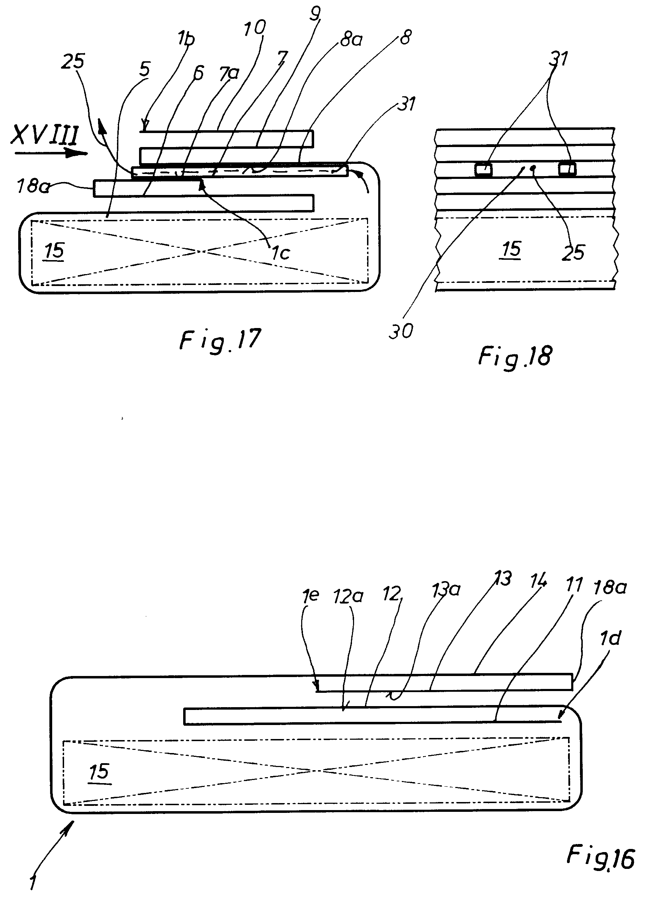

In den Fig. 16 bis 18 ist eine weitere luftdurchlässige Zone in Form eines Luftkanals 30 offenbart, der von zwei Abstandshaltern 31 in Verbindung mit den Bereichen der daran angrenzenden Faltlagen, hier 7 und 8 gemäß Fig. 5 in Verbindung mit Fig. 17, gebildet wird. Der Kanal 30 führt - wie anschaulich aus den Fig. 17 und 18 hervorgeht - bis in den Innenraum 15 des zusammengefalteten Tischtuches 1. Die mit den Fig. 6 und 13 übereinstimmenden Teile sind in Fig. 16 mit identischen Bezugsziffern versehen. Durch die eingelegten Abstandshalterstäbe 31 werden beim Evakuierungsvorgang die Kontaktflächen 7a und 8a der Faltlagen 7 und 8 auf Abstand auseinandergehalten und können durch den "Saugdruck" nicht mehr miteinander "verkleben".

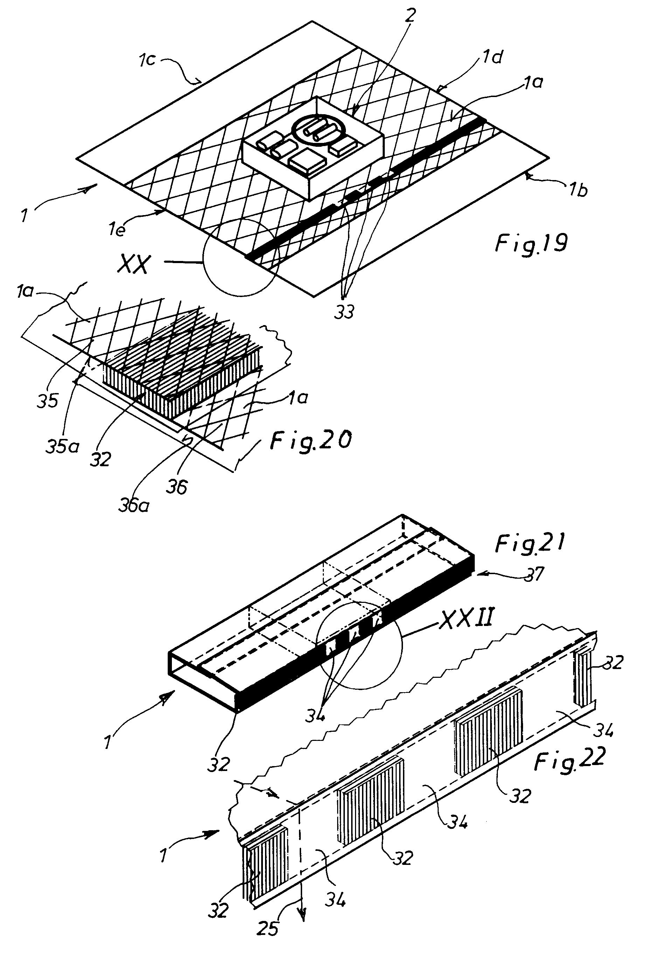

In den Fig. 19 bis 22 ist eine weitere Ausführungsform des Tischtuches 1 dargestellt, wobei eine zu einer Längsseite 1b des Tischtuches 1 parallel verlaufende Klebeschicht 32 mit Unterbrechungen 33 vorgesehen ist. Die Unterbrechungen 33 der Klebeschicht 32 bilden mit den angrenzenden Deckflächen der zunächst geschnittenen und dann mit der Klebeschicht 32 wieder miteinander verbundenen Teilen 35, 36 der Polyethylenfolie des Tischtuches 1 Strömungskanäle 34, um während des Evakuierungsvorganges Luft aus dem Innenraum 15 des eingeschlagenen Tischtuches 1 nach außen gelangen zu lassen.

Wie die Fig. 20 in Verbindung mit Fig. 19 zeigt, wird zur geschickten Anbringung der Klebeschicht 32 die Polyethylenfolie des Tischtuches 1 in die beiden Teile 35 und 36 getrennt und sodann durch die Klebeschicht 32 mit ihren Unterbrechungen 33 wieder zusammengeklebt, wodurch die Kanäle 34 von den Unterbrechungen 33 der Klebeschicht 32 und den angrenzenden Flächen 35a, 36a der geschnittenen Teile 35, 36 der Polyethylenfolie des Tischtuches 1 gebildet werden. Auf diese Weise können die einen Teil der Kanäle 34 bildenden Unterbrechungen 33 während des Produktionsvorganges des Tischtuches 1 kontinuierlich in dieser eingebracht werden.

Dabei ist es gemäß den Fig. 21 und 22 von besonderem Vorteil, wenn die Klebeschicht 32 mit den Kanälen 34 nach dem Einschlag der Längsseiten 1b und 1c des Tischtuches 1 in der Nähe einer Längsschmalseite 37 angeordnet ist. Auf diese Weise wird dann sämtlichen Anforderungen genügende Durchströmungskanäle 34 gewährleistet.

| **Bezugszeichenliste:** | |
|---|---|
| Tischtuch | 1 |
| Vliesstoff | 1a |
| Längsseiten des Tischtuches 1 | 1b, 1c |
| Schmalseiten des Tischtuches 1 | 1d, 1e |
| Operations-Set-Komponenten | 2 |
| Richtungspfeile der Einschlagrichtung | 3, 4 |
| Faltlagen | 5, 6, 7, 8, 9, 11, 12, 13, 14 |
| Kontaktflächen der Faltlagen 7, 8; 12, 13 | 7a, 8a; 12a, 13a |
| Innenraum des Tischtuches 1 | 15 |
| luftdurchlässige Zone | 16 |
| Membran | 17 |
| Außenrandbereich der Membran 17 | 17a |
| Außenseite des gefalteten Tischtuches 1 | 18 |
| Faltkante | 18a |
| Öffnung in einer Außenseite 18 | 19 |
| Öffnungsrand | 19a |
| Schlitze | 20 |
| Schlitzlängsseite | 20a |
| Kunststoffolie | 21 |
| Klebestreifen der Kunststoffolie | 22, 23, 24 |
| Strömungspfeile der Luft | 25 |
| Verpackung | 26 |
| Deckel der Verpackung 26 | 27 |
| Abziehverschluß (Peel-Verschluß) | 28 |
| Schlauchleitung | 29 |
| Strömungskanäle der Luft | 30, 34 |
| Abstandshalter | 31 |
| Klebeschicht | 32 |
| Unterbrechungen der Klebeschicht 32 | 33 |
| Teile der geschnittenen Polyethylenfolie des Tischtuches 1 | 35, 36 |
| der Klebeschicht 32 zugekehrte Flächen von 35, 36 | 35a, 36a |

## Patentansprüche

1. Tischtuch (back table cover) für einen OP-Beistelltisch mit darin eingeschlagenen, unterschiedlichen Operations-Set-Komponenten, wie mayo-stand-covers, OP-Abdecktüchern, Klebestreifen, Handtüchern, Strümpfen oder dergleichen, welches von einer Verpackung umhüllt ist, **dadurch gekennzeichnet,** daß das zusammengefaltete Tischtuch (1) an mindestens einer seiner Außenbereiche (18, 18a) mit einer zu seinem Innenraum (15) führenden, luftdurchlässigen Zone (17, 20, 29, 30, 34) versehen ist.

2. Tischtuch nach Anspruch 1, **dadurch gekennzeichnet,** daß die luftdurchlässige Zone (17, 20, 29, 30, 34) an einer Faltkante (18a) des Tischtuches (1) vorgesehen ist.

3. Tischtuch nach Anspruch 1, **dadurch gekennzeichnet,** daß die luftdurchlässige Zone (17, 20, 29, 30, 34) an einer Außenseite (18) des Tischtuches (1) angeordnet ist.

4. Tischtuch nach Anspruch 1, **dadurch gekennzeichnet,** daß die luftdurchlässige Zone (29, 30) zwischen zwei Faltflächen (12a, 13a) des Tischtuches (1) vorgesehen ist.

5. Tischtuch nach einem oder mehreren der Ansprüche 1 bis 4, **dadurch gekennzeichnet,** daß die luftdurchlässige Zone aus in das Tischtuch (1) eingebrachten Schlitzen (20) besteht, die von einer Kunststoffolie (21) abgedeckt sind.

6. Tischtuch nach Anspruch 5, **dadurch gekennzeichnet,** daß die Kunststoffolie (21) an den beiden Enden eines jeden Schlitzes (20) sowie an der dem Innenbereich des Tischtuches (1) zugeordneten Schlitzlängsseite (20a) mit dem Tischtuch (1) verklebt, verschweißt oder durch eine Prägung verbunden ist.

7. Tischtuch nach einem oder mehreren der Ansprüche 1 bis 4, **dadurch gekennzeichnet,** daß die luftdurchlässige Zone aus einer in eine Außenseite (18) des gefalteten Tischtuches (1) eingebrachten oder aus einer bis zu einer Faltkante (18a) reichenden Öffnung (19) besteht, die von einer Membran (17) verschlossen ist, welche von innen nach außen sowie umgekehrt luftdurchlässig, hingegen in umgekehrter Richtung flüssig- und feststoffdicht ist.

8. Tischtuch nach Anspruch 7, **dadurch gekennzeichnet,** daß die Membran (17) aus einem Tyvek- oder einem anderen Vliesstoff oder einem Tissue-Material gebildet ist.

9. Tischtuch nach Anspruch 7 oder 8, **dadurch gekennzeichnet,** daß die Membran (17) an ihren Außenrandbereichen (17a) dicht mit dem Rand (19a) der Öffnung (19) des Tischtuches (1) verklebt, verschweißt oder verprägt ist.

10. Tischtuch nach einem oder mehreren der Ansprüche 1 bis 4, **dadurch gekennzeichnet,** daß die luftdurchlässige Zone aus einem Luftkanal (29, 30) besteht, der entweder von einer eingelegten, bis in den Innenraum (15) des gefalteten Tischtuches (1) reichenden Schlauchleitung (29) oder von einem von Abstandshaltern (31) zwischen den Flächen (7a, 8a) der Faltlagen (7, 8) und bis in den Innenraum (15) führenden Kanal (30) gebildet ist.

11. Tischtuch nach einem oder mehreren der Ansprüche 1 bis 4, **dadurch gekennzeichnet,** daß die luftdurchlässige Zone aus einem Luftkanal (34) besteht, der von mindestens einer Unterbrechung (33) einer zu einer Längsseite (1b oder 1c) des Tischtuches (1) parallel verlaufenden Klebeschicht (32) in Verbindung mit den an die Unterbrechung (33) angrenzenden Flächen (7a, 8a) der jeweiligen Faltlagen (7, 8) gebildet ist.

12. Tischtuch nach Anspruch 11, **dadurch gekennzeichnet,** daß die Klebeschicht (32) des Tischtuches (1) zwischen zwei getrennten und von der Klebeschicht (32) wieder verbundenen Kunststoffolien-Teilen (35, 36) angeordnet ist.

13. Tischtuch nach einem oder mehreren der Ansprüche 1 bis 12, **dadurch gekennzeichnet,** daß es in einer Vakuumverpackung (26, 27) angeordnet ist.

14. Tischtuch nach einem oder mehreren der Ansprüche 1 bis 13, **dadurch gekennzeichnet,** daß die Vakuumverpackung (26, 27) an mindestens zwei Seitenkanten einen Abzieh-Verschluß (28) (Peel-Verschluß) aufweist.
